(19) Europäisches Patentamt
European Patent Office
Office européen des brevets

(11) Veröffentlichungsnummer: **0 345 581 A2**

(12) # EUROPÄISCHE PATENTANMELDUNG

(21) Anmeldenummer: **89109611.7**

(22) Anmeldetag: **27.05.89**

(51) Int. Cl.4: **C08F 2/44 , A61K 6/08**

(30) Priorität: **10.06.88 DE 3819777**

(43) Veröffentlichungstag der Anmeldung:
**13.12.89 Patentblatt 89/50**

(84) Benannte Vertragsstaaten:
**AT BE CH DE ES FR GB GR IT LI LU NL SE**

(71) Anmelder: **BAYER AG**
**D-5090 Leverkusen 1 Bayerwerk(DE)**

(72) Erfinder: **Podszun, Wolfgang, Dr.**
**Roggendorfstrasse 55**
**D-5000 Köln 80(DE)**
Erfinder: **Mazanek, Jan, Dr.**
**Haferkamp 2**
**D-5000 Köln 80(DE)**
Erfinder: **Reiners, Jürgen, Dr.**
**Carl-Rumpff-Strasse 57**
**D-5090 Leverkusen 1(DE)**
Erfinder: **Winkel, Jens, Dr.**
**Letterhaus-Strasse 1**
**D-5000 Köln 71(DE)**
Erfinder: **Heid, Renate, Dr.**
**Buchenweg 2**
**D-6803 Edingen-Neckarhausen(DE)**

(54) **Füllstoffhaltige, polymerisierbare Massen und deren Verwendung.**

(57) Neue polymerisierbare Massen, die zur Herstellung biokompatibler Werkstoffe für medizinische und zahnmedizinische Anwendungen verwendet werden können enthalten

a) 15 bis 60 Gew.-% Monomere mit radikalisch polymeri sierbaren Doppelbindungen,

b) 8 bis 40 Gew.-% faserförmige, anorganische Füllstoffe mit einer mittleren Faserlänge von 10 nm bis 300 µm und einem Verhältnis von Länge zu Durchmesser von 2:1 bis 300:1,

c) 15 bis 60 Gew.-% nicht-faserförmige, anorganische Füllstoffe mit einer mittleren Teilchengröße von 10 nm bis 200 µm und

d) 0 bis 10 Gew.-% Hilfsmittel.

EP 0 345 581 A2

## Füllstoffhaltige, polymerisierbare Massen und deren Verwendung

Die vorliegende Erfindung betrifft füllstoffhaltige, polymerisierbare Massen auf der Basis von Monomeren und faserförmigen und nicht-faserförmigen anorganischen Füllstoffen.

Füllstoffhaltige, polymerisierbare Massen, die unter Formgebung ausgehärtet werden können, haben als Werkstoffe vielseitige Anwendungen auf technischem und medizinischem Gebiet. Der Füllstoff kann dabei die polymerisierbare Masse in unterschiedlicher Weise beeinflussen. So kann er das Monomere verdünnen und auf diese Weise den Polymerisationsschrumpf und die Polymerisationswärme herabsetzen; er kann das Monomere auch verdicken und damit der Gesamtmischung eine für die Verarbeitung günstige Konsistenz verleihen; er kann die physikalischen Eigenschaften des Systems verändern und er bietet in der Regel ökonomische Vorteile.

Für medizinische Einsatzzwecke, z.B. bei Dentalmaterialien und Knochenzementen, werden im allgemeinen Füll stoffe mit kugelförmiger oder unregelmäßiger Gestalt eingesetzt. Solche Massen zeichnen sich einerseits durch eine hohe Steifigkeit, einen hohen Elastizitätsmodul, hohe Druckfestigkeit und hohe Härte aus, erreichen aber hinsichtlich ihrer Zugfestigkeit und ihrer Zähigkeit nicht immer die gewünschten Werte. Composites mit höheren Zugfestigkeiten können bekanntermaßen durch die Verwendung von faserhaltigen Füllstoffen erhalten werden. In der Arbeit von S. Parl et al., Biomedical Engineering 4, Recent Developments, Proceedings of the Southern Biomedical Engineering Conference 4th, Jackson Miss., 11.-12.10.1985, sind Composites, die mit silanisierten Glasfasern gefüllt sind, beschrieben. Ein Nachteil dieses Systems besteht jedoch darin, daß der erreichbare Volumenfüllgrad mengenmäßig begrenzt ist. Infolgedessen weisen diese Massen einen unerwünscht hohen Polymerisationsschrumpf auf.

Es wurden nun polymerisierbare Massen gefunden, die dadurch gekennzeichnet sind, daß sie

a) 15 bis 60 Gew.-% Monomere mit radikalisch polymerisierbaren Doppelbindungen,

b) 8 bis 40 Gew.-% faserförmige, anorganische Füllstoffe mit einer mittleren Faserlänge von 10 nm bis 300 µm und mit einem Verhältnis von Länge zu Durchmesser von 2:1 bis 300:1,

c) 15 bis 60 Gew.-% nicht-faserförmige, anorganische Füllstoffe mit einer mittleren Teilchengröße von 10 nm bis 200 µm und

d) 0 bis 10 Gew.-% Hilfsmittel

enthalten,

wobei die Summe a) plus b) plus c) plus d) 100 Gew.-% ergibt.

Die hier und in folgenden angegebenen Gewichtsprozente beziehen sich auf die gesamte erfindungsgemäße Masse, soweit nicht etwas anderes angegeben ist.

Bevorzugte erfindungsgemäße polymerisierbare Massen enthalten

15 bis 45 Gew.-% der Komponente a),

15 bis 40 Gew.-% der Komponente b),

15 bis 40 Gew.-% der Komponente c) und

0,1 bis 5 Gew.-% der Komponente d).

Als Monomere mit radikalisch polymerisierbaren Doppelbindungen (Komponente a)) eignen sich beispielsweise unter Verarbeitungsbedingungen flüssige Vinyl- und Vinylidenverbindungen, wie Styrol, α-Methylstyrol, Vinylacetat, Acrylnitril und Ester der Acryl- und der Methacrylsäure. Besonders geeignet sind Acryl- und Methacrylsäurealkylester mit $C_1$ bis $C_{12}$ Alkoholen, insbesondere Methyl(meth)acrylat, Ethyl(meth)acrylat, n-Propyl(meth)acrylat, i-Propyl(meth)acrylat und Ethylhexyl (meth)acrylat. Unter der Bezeichnung (Meth)acrylat werden hier und im folgenden sowohl die Acrylsäureester als auch die entsprechenden Methacrylsäureester verstanden.

Weitere bevorzugte Monomere mit radikalisch polymerisierbaren Doppelbindungen sind Ester der Acrylsäure oder Methacrylsäure mit zwei oder mehr, beispielsweise 2 bis 8, Doppelbindungen im Molekül. Als Beispiele seien genannt: Ethylenglykoldi(meth)acrylat, Diethylenglykoldi(meth)acrylat, Triethylenglykol(meth)acrylat, Hexandioldi(meth)acrylat, Glycerindi(meth)acrylat, Glycerintri(meth)acrylat, Trimethylolpropantri(meth)acrylat, Pentaerythritdi(meth)acrylat, Pentaerythrittri(meth)acrylat, Pentaerythrittetra(meth)acrylat, Dipentaerythrithexa(meth)acrylat und Derivate von Bisphenol-A, wie Bisphenol-A-di(meth)acrylat, Bisphenol-A-diglykoldi(meth)acrylat und Bisphenol-A-diglycidyldi(meth)acrylat.

Weitere bevorzugte Monomere mit radikalisch polymerisierbaren Doppelbindungen sind Derivate des Tricyclodecans wie sie in der EP-OS 0 023 686 beschrieben sind und Urethan(meth)acrylate, die beispielsweise durch Umsetzung von Diisocyanaten und Hydroxyalkyl(meth)acrylaten zugänglich sind.

Weitere bevorzugte Monomere mit radikalisch polymerisierbaren Doppelbindungen entsprechen der Formel (I)

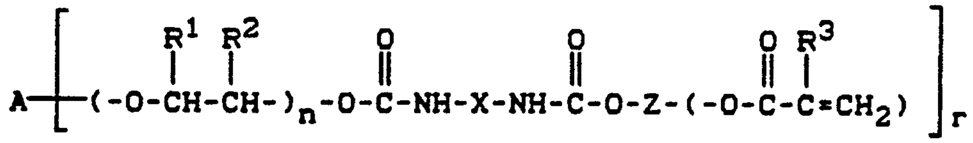

(I),

in der

A ein geradkettiger oder verzweigter, gegebenenfalls 1 bis 3 Sauerstoffbrücken enthaltender, aliphatischer Rest mit 2 bis 20 C-Atomen, ein aromatischer Rest mit 6 bis 24 C-Atomen, ein araliphatischer Rest mit 7 bis 26 C-Atomen oder ein cycloaliphatischer Rest mit 6 bis 26 C-Atomen ist,

r für die Anzahl der von A ausgehenden Ketten steht und eine Zahl von 2 bis 6 bedeutet,

$R^1$ Wasserstoff und

$R^2$ Wasserstoff oder Methyl bedeuten,

n für jede von A ausgehende Kette unabhängig eine ganze Zahl von 0 bis 5 bedeutet,

X ein zweibindiger, geradkettiger oder verzweigter aliphatischer Rest mit 2 bis 24 C-Atomen, ein aromatischer Rest mit 6 bis 26 C-Atomen, ein araliphatischer Rest mit 7 bis 26 C-Atomen oder ein cycloaliphatischer Rest mit 6 bis 26 C-Atomen bedeutet, wobei die aliphatischen, aromatischen, araliphatischen und/oder cycloaliphatischen Reste 1 oder 2 Sauerstoffbrücken enthalten können, und wobei mehrere der aliphatischen, aromatischen araliphatischen und/oder monocycloaliphatischen Reste über gegebenenfalls substituierte Methylengruppen verbunden sein können,

Z einen zweibindigen, geradkettigen oder verzweigten aliphatischen Kohlenwasserstoffrest mit 3 bis 15 C-Atomen, der gegebenenfalls 1 bis 3 Sauerstoffbrücken enthält und gegebenenfalls durch 1 bis 4 zusätzliche (Meth)acrylatreste substituiert sein kann, bedeutet, und

$R^3$ für jede von A ausgehende Kette unabhängig Wasserstoff oder Methyl bedeutet.

Soweit A, X und Z für aliphatische und/oder cycloaliphatische Reste stehen, handelt es sich dabei vorzugsweise um gesättigte Reste.

In Formel (I) bedeutet A vorzugsweise einen r-wertigen, gesättigten, aliphatischen Rest mit 3 bis 10 C-Atomen, beispielsweise

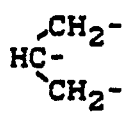 , 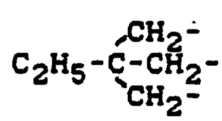 oder 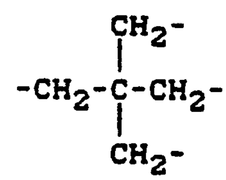 ,

bedeutet n vorzugsweise eine ganze Zahl von 0 bis 2, bedeutet X vorzugsweise einen gesättigten, aliphatischen Rest mit 2 bis 12 C-Atomen oder einen gesättigten, cycloaliphatischen Rest mit 6 bis 18 C-Atomen und bedeu tet Z vorzugsweise einen zweibindigen, geradkettigen oder verzweigten Rest mit 2 bis 8 C-Atomen, beispielsweise $-CH_2-CH_2-$ oder

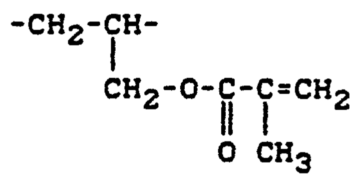

In vielen Fällen ist es vorteilhaft, Mischungen unterschiedlicher Monomerer, z.B. Mischungen aus monofunktionellen und polyfunktionellen Monomeren einzusetzen. Durch Variation der qualitativen und/oder quantitativen Zusammensetzung solcher Mischungen kann die Monomerviskosität in einem breiten Bereich variiert werden.

Als faserförmige, organische Füllstoffe (Komponente b)) eignen sich insbesondere solche auf der Basis von Gläsern, Quarz, Siliciumdioxid, Silikaten, Alumosilikaten und Aluminiumoxid, sowie Siliciumcarbid-Whiskers, Siliciumnitrid-Whiskers, Wollastonit und Kalziumsulfat. Glasfasern können vorzugsweise Durchmesser von 0,1 bis 10 µm, und Längen von 0,5 bis 1000 µm, besonders bevorzugt Durchmesser von 0,2

bis 2,0 µm und Längen von 1 bis 300 µm aufweisen. Gut geeignet sind auch anorganische Microfasern und Whiskers, besonders solche mit einem Gehalt an Siliciumdioxid und/oder Aluminiumoxid. Die bevorzugten Längen dieser Whiskers liegen im Bereich von 0,05 bis 5 µm, die bevorzugten Durchmesser im Bereich von 0,005 bis 0,5 µm. Erfindungsgemäße Massen mit besonders guten mechanischen Eigenschaften lassen sich erhalten, wenn man Mischungen aus Fasern und/oder Whiskers unterschiedlicher Länge, beispielsweise Microglasfasern in Kombination mit Whiskers, verwendet.

Die faserförmigen, anorganischen Füllstoffe gelangen vorzugsweise in mit Haftvermittlern behandelter Form zum Einsatz. Als Haftvermittler eignen sich beispielsweise Silan- und Titanatverbindungen, wie Trimethylchlorsilan, Hexamethyldisiloxan, 3-Aminopropyltrimethoxysilan, Butyltitanat und Isopropyltitanat. Besonders gut geeignet sind Haftvermittler mit polymerisierbaren Gruppen, wie Vinyltrimethylsiloxan, Allyltrimethoxysilan und γ-Methacryloyloxypropyltrimethoxysilan.

Die nicht-faserförmigen, anorganischen Füllstoffe (Komponente c)) können z.B. au kugelig, annähernd kugelig oder durch Mahlung oder Herstellung (z.B. in flammhydrolytischen Verfahren hergestellte) erzeugten, regelmäßig oder unregelmäßig geformten Partikeln bestehen. Die nicht-faserförmigen, anorganischen Füllstoffe können beispielsweise mit einer bimodalen oder polymodalen Teilchengrößenverteilung eingesetzt werden. So sind Füllstoffe mit einem ersten Maximum in der Verteilungskurve im Bereich von 10 bis 800 nm und einem zweiten Maximum im Bereich von 0,5 bis 50 µm besonders bevorzugt. Geeignete nicht-faserförmige, anorganische Füllstoffe sind beispielsweise Gläser in Form von Kugeln oder in Form von durch Mahlung gewonnenen, unregelmäßig geformten Partikeln mit mittleren Teilchengrößen im Bereich von 0,5 bis 50 µm. Des weiteren geeignet sind flammhydrolytisch gewonnene Oxide des Siliciums und Aluminiums mit Teilchengrößen im Bereich von 10 bis 500 nm. Auch die nicht-faserförmigen, anorganischen Füllstoffe gelangen vorzugsweise in mit Haftvermittlern behandelter Form zum Einsatz. Als Haftvermittler eignen sich dabei z.B. die bei der Komponente b) beschriebenen.

Gegebenenfalls kann die erfindungsgemäße Masse Hilfsmittel enthalten (Komponente d)), beispielsweise insgesamt 0,1 bis 10 Gew.-% Hilfsmittel. Unter Hilfsmitteln sind beispielsweise Initiatoren, Stabilisatoren, Lichtschutzmittel, Pigmente, Farbstoffe, Fluoreszenzmittel und/oder Weichmacher zu verstehen.

Als Initiatoren können beispielsweise Radikale bindende Systeme wie Peroxide oder aliphatische Azoverbindungen angewendet werden, beispeilsweise Benzoylperoxid, Laurylperoxid, Cumolhydroperoxid, Cyclohexylpercabonat oder Azodiisobuttersäuredinitril. Initiatoren kommen bevorzugt in Mengen von 0,1 bis 2,5 Gew.-% zum Einsatz. Die radikalische Polymerisation kann bei erhöhter Temperatur im allgemeinen allein initiiert durch Peroxide oder andere Radikalbildner durchgeführt werden. Soll die radikalische Polymerisation bei Raumtemperatur durchgeführt werden, so ist im allgemeinen ein Zusatz von Beschleunigern, z.B. aromatischen Aminen, vorteilhaft. Geeignete Beschleuniger sind z.B. N,N-substituierte Toluidine und Xylidine, wie N,N-Dimethyl-p-toluidin oder N,N-Bis(2-hydroxy-ethyl)-xylidin. Gute Aushärtungszeiten erzielt man im allgemeinen mit Aminzusätzen von 0,5 bis 3 Gew.-%.

Es ist jedoch auch möglich, erfindungsgemäße Massen unter Einwirkung von Licht zu polymerisieren, beispielsweise unter Einwirkung von UV-Licht, sichtbarem Licht oder Laserlicht. In diesem Fall enthalten erfindungsgemäße Massen vorzugsweise Photopolymerisationsinitiatoren und gegebenenfalls Beschleuniger dafür.

Als Photopolymerisationsinitiatoren können beispielsweise Carbonylverbindungen verwendet werden, wie Benzoin und dessen Derivate, insbesondere Benzoinmethylether, Benzil und Benzilderivate, beispielsweise 4,4-Oxidibenzil oder andere α-Dicarbonylverbindungen, z.B. Diacetyl, 2,3-Pentadion oder Metallcarbonyle, 1,2-Chinone oder deren Derivate. Der Anteil an solchen Photopolymerisationsinitiatoren beträgt vorzugsweise 0,01 bis 5 Gew.-%.

Als Beschleuniger für die Photopolymerisation sind beispielsweise aromatische Amine wie p-Toluidin und Dimethyl-p-toluidin, Trialkylamine wie Trihexylamin, Polyamine, wie N,N,N′,N′-Tetraalkylalkylendiamin, Barbitursäure, Dialkylbarbitursäuren und/oder Sulfimide verwendbar, vorzugsweise in Mengen von 0,1 bis 5 Gew.-%.

Um ein Nachdunkeln während des Alterns zu vermeiden, kann es zweckmäßig sein, den erfindungsgemäßen Massen UV-Stabilisatoren zuzusetzen. Bevorzugte UV-Stabilisatoren sind z.B. 2-Hydroxy-4-methoxybenzophenon und 2-(2′-Hydroxy-5′-methylphenyl)-benzotriazol. Prinzipiell ist jedes UV-absorbierende Agens, insbesondere wenn es physiologisch inert ist, als Additiv für erfindungsgemäße Massen geeignet. Beispielhaft seien noch genannt: Hydrochinon, p-Benzochinon und p-Butylhydroxytoluol. p-Butylhydroxytoluol kann in ausgehärteten erfindungsgemäßen Massen auch als Antioxidans wirken. UV-Stabilisatoren können in erfindungsgemäßen Massen, beispielsweise in Mengen von 0,01 bis 1,0 Gew.-%, vorhanden sein.

Zur Einfärbung erfindungsgemäßer Massen können übliche Pigmente und Farbstoffe eingesetzt werden, beispielsweise in Mengen von 0,05 bis 2 Gew.-%.

Erfindungsgemäße Massen lassen sich zu Formkörpern, insbesondere für technische, medizinische und zahnmedizinische Anwendungen aushärten. Die gehärteten Formkörper zeichnen sich durch eine günstige Eigenschaftskombination aus. Sie besitzen eine hohe Steifigkeit, Härte und Abrasionsbeständigkeit verbunden mit guter Zugfestigkeit und Zähigkeit. Außerdem tritt während der Härtung nur ein geringer Schrumpf auf.

Erfindungsgemäße füllstoffhaltige, polymerisierbare Massen können hergestellt werden, indem man die Komponenten a) bis d) miteinander vermischt, wobei im allgemeinen gut handhabbare Pasten entstehen. Das Mischen kann im allgemeinen bei Raumtemperatur ohne zu heizen oder zu kühlen vorgenommen werden. Höhere Temperaturen, z.B. solche über 40 °C sind normalerweise zu vermeiden, weil sonst die Gefahr einer vorzeitigen Polymerisation besteht. Bei sehr hochviskosen Mischungen kann es deshalb zweckmäßig sein, die Knetwärme durch Kühlung abzuführen.

Die vorliegende Erfinding betrifft weiterhin die Verwendung von füllstoffhaltigen, polymerisierbaren Massen, die

a) 15 bis 60 Gew.-% Monomere mit radikalisch polymerisierbaren Doppelbindungen,

b) 8 bis 40 Gew.-% faserförmige, anorganische Füll stoffe mit einer mittleren Faserlänge von 10 nm bis 300 µm und einem Verhältnis von Länge zu Durchmesser von 2:1 bis 300:1,

c) 15 bis 60 Gew.-% nicht-faserförmige, anorganische Füllstoffe mit einer mittleren Teilchengröße von 10 nm bis 200 µm und

d) 0 bis 10 Gew.-% Hilfsmittel

enthalten, wobei die Summe a) plus b) plus c) plus d) 100 Gew.-% ergibt,
zur Herstellung von biokompatiblen Werkstoffen für medizinische und zahnmedizinische Anwendungen.

## Beispiele

### Beispiel 1 (nicht erfindungsgemäß)

Herstellung eines Monomeren (Komponente a))

45,6 g handelsübliches Glycerindimethacrylat, 0,1 g Dibutylzinndilaurat und 34 mg Jonol wurden in 50 ml getrocknetem Aceton gelöst und zu 33,6 g Hexamethylendiisocyanat bei 45 bis 50 °C zugetropft. Bei dieser Temperatur wurde gerührt, bis die Hälfte der NCO-Gruppen abreagiert hatte. Dann wurden weitere 6,8 g Pentaerythrit in 50 ml Aceton zugegeben. Die Mischung wurde 48 h bei 50 °C gerührt, danach war im IR-Spektrum kein Isocyanat mehr nachweisbar. Das Reaktionsprodukt wurde über Aktivkohle filtriert und vom Lösungsmittel befreit. Das erhaltene Produkt wies eine Molmasse von 1620 auf (osmotisch bestimmt).

### Beispiel 2

18,7 g des gemäß Beispiel 1 hergestellten Monomers, 15,3 g 1,6-Hexandioldiacrylat, 29 g Glasfasern (mittlerer Durchmesser 7 µm, mittlere Länge 150 µm, silanisiert mit γ-Methacryloyloxypropyltrimethoxysilan), 47,6 g hochdisperses Siliciumdioxid (mittlere Korngröße 50 nm, silanisiert mit γ-Methacryloyloxypropyltrimethoxysilan), 170 mg Azoisobuttersäuredintril und 13 mg 2,6-Di-tert.-butyl-4-methylphenol wurden in einem Duplexmischer im Verlauf von 30 Min. zu einer Paste geknetet. Die erhaltene Paste wurde anschließend innerhalb von 20 Min. bei 140 °C und 200 bar zu Prüfkörpern ausgehärtet. An diesen wurden folgende Werte bestimmt (die angegebenen Prüfmethoden wurden bei allen Beispielen angewendet):

| | | | |
|---|---|---|---|
| Biegefestigkeit | (DIN 13922) | : | 138 ± 7,8 N/mm² |
| Biege-E-Modul | (DIN 13922) | : | 12800 ± 250 N/mm² |
| Schlagzähigkeit | (Dynstatprüfung nach DIN 13922) | : | 5,2 ± 0,2 kJ/m² |

### Beispiel 3

16,5 g des gemäß Beispiel 1 hergestellten Monomers, 14,5 g 1,6-Hexandioldimethacrylat, 20 g silanisierte Glasfasern (mittlerer Durchmesser 0,6 µm, mittlere Länge 20 µm), 10 g silanisierte $SiO_2$-Whiskers (mittlerer Durchmesser 40 nm, mittlere Länge 0,8 µm), 30 g silanisierter gemahlener Glasfüllstoff (Schott GM 32087, mittlerer Durchmesser 2,5 µm), 20 g silanisiertes hochdisperses Siliciumdioxid (mittlerer Durchmesser 50 nm), 150 mg Azoisobuttersäuredinitril und 10 mg 2,6-Di-tert.-butyl-4-methylphenol wurden in einem Duplexmischer innerhalb von 30 Min. zu einer Paste geknetet.

Die Paste wurde innerhalb von 20 Min. bei 140 °C und 200 bar gehärtet. Dann wurden folgende Werte bestimmt:

| | | |
|---|---|---|
| Biegefestigkeit | : | 152,3 ± 13,5 $N/mm^2$ |
| Biege-E-Modul | : | 15800 ± 630 $N/mm^2$ |
| Schlagzähigkeit | : | 6,4 ± 0,5 $kJ/m^2$ |

Beispiel 4

30 g Bisphenol-A-diglycidyldimethacrylat, 18 g Triethylenglykoldimethacrylat, 12 g silanisierte Kalziumsilikat-Whiskers (mittlerer Durchmesser 0,2 µm, mittlere Länge 5 µm), 27 g silanisierte Glasfasern (mittlerer Durchmesser 0,6 µm, mittlere Länge 20 µm), 63 g silanisiertes hochdisperses Siliciumdioxid (mittlerer Durchmesser 0,05 µm), 0,1 g Campherchinon, 0,25 g N-Dimethylaminoethylmethacrylat und 10 mg 2,6-Di-tert.-butyl-4-methylphenol wurden in einem Duplexmischer innerhalb von 30 Min. zu einer Paste geknetet.

Diese Paste wurde in einem Lichtofen (Dentacolor XS, Fa. Kulzer) innerhalb von 90 Sek. zu Prüfkörpern ausgehärtet. Daran wurden folgende Werte bestimmt:

| | | |
|---|---|---|
| Beigefestigkeit | : | 141 ± 9,0 $N/mm^2$ |
| Biege-E-Modul | : | 13500 ± 320 $N/mm^2$ |
| Schlagzähigkeit | : | 6,1 ± 0,4 $kJ/m^2$ |

Beispiel 5

62 g Bisphenol-A-diglycidyldimethacrylat, 38 g Triethylenglykoldimethacrylat, 160 g silanisierte $SiO_2$-Whiskers (mittlerer Durchmesser 40 nm, mittlere Länge 20 µm), 35 g silanisiertes hochdisperses Siliciumdioxid (mittlerer Durchmesser 50 nm), 0,5 g Dibenzoylperoxid und 50 mg 2,6-Di-tert.-butyl-4-methyl-phenol wurden zu einer Paste geknetet.

Die Paste wurde in 20 Min. bei 140 °C und 200 bar ausgehärtet. Dann wurden daran folgende Werte bestimmt:

| | | |
|---|---|---|
| Biegefestigkeit | : | 155,3 ± 13,5 $N/mm^2$ |
| Biege-E-Modul | : | 14650 ± 650 $N/mm^2$ |
| Schlagzähigkeit | : | 7,3 ± 0,8 $kJ/m^2$ |

Beispiel 6

62 g Bisphenol-A-diglycidyldimethacrylat, 38 g Triethylenglykoldimethacrylat, 86 g silanisierte Glasfasern (mittlerer Durchmesser 0,5 µm, mittlere Länge 10 µm), 68 g silanisiertes hochdisperses Siliciumdioxid (mittlerer Durchmesser 0,05 µm), 0,2 g Campherchinon, 0,5 g N,N-Dimethylaminoethylmethacrylat und 0,1 g 2,6-Di-tert.-butyl-4-methyl-phenol wurden zu einer Paste geknetet.

Die Paste wurde in einem Lichtofen (Dentacolor XS, Fa. Kulzer) innerhalb von 90 Sek. zu Prüfkörpern ausgehärtet. An diesen wurden folgende Werte bestimmt:

| | | |
|---|---|---|
| Biegefestigkeit | : | 132,1 ± 11,3 N/mm² |
| Biege-E-Modul | : | 9700 ± 310 N/mm² |
| Schlagzähigkeit | : | 5,4 ± 0,5 kJ/m² |

## Ansprüche

Polymerisierbare Massen, dadurch gekennzeichnet, daß sie
a) 15 bis 60 Gew.-% Monomere mit radikalisch polymerisierbaren Doppelbindungen,
b) 8 bis 40 Gew.-% faserförmige, anorganische Füllstoffe mit einer mittleren Faserlänge von 10 nm bis 300 µm und miteinem Verhältnis von Länge zu Durchmesser von 2:1 bis 300:1,
c) 15 bis 60 Gew.-% nicht-faserförmige, anorganische Füllstoffe mit einer mittleren Teilchengröße von 10 nm bis 200 µm und
d) 0 bis 10 Gew.-% Hilfsmittel
enthalten, wobei die Summe a) plus b) plus c) plus d) 100 Gew.-% ergibt.

2. Polymerisierbare Massen gemäß Anspruch 1, dadurch gekennzeichnet, daß sie

| | |
|---|---|
| 15 bis 45 Gew.-% | der Komponente a), |
| 15 bis 40 Gew.-% | der Komponente b), |
| 15 bis 40 Gew.-% | der Komponente c) und |
| 0,1 bis 5 Gew.-% | der Komponente d), |

enthalten.

3. Polymerisierbare Massen gemäß Ansprüchen 1 und 2, dadurch gekennzeichnet, daß sie als Monomere mit radikalisch polymerisierbaren Doppelbindungen Ester der Acrylsäure oder Methacrylsäure mit 2 bis 8 Doppelbindungen enthalten.

4. Polymerisierbare Massen nach Ansprüchen 1 bis 3, dadurch gekennzeichnet, daß sie als Komponente a) Mischungen unterschiedlicher Monomere enthalten.

5. Polymerisierbare Massen nach Ansprüchen 1 bis 4, dadurch gekennzeichnet, daß sie als faserförmige, anorganische Füllstoffe solche auf der Basis von Gläsern, Quarz, Siliciumdioxid, Silikaten, Alumosilikaten, Aluminiumoxid, Siliciumcarbid-Whiskern, Silciumnitrid-Whiskern, Wollastonit und/oder Kalziumsulfat enthalten.

6. Polymerisierbare Massen nach Ansprüchen 1 bis 5, dadurch gekennzeichnet, daß die faserförmigen, anorganischen Füllstoffe in mit Haftvermittlern behandelter Form zum Einsatz gelangen.

7. Polymerisierbare Massen nach Ansprüchen 1 bis 6, dadurch gekennzeichnet, daß die nicht-faserförmigen, anorganischen Füllstoffe aus kugeligen, annähernd kugeligen oder durch Mahlung oder Herstellung erzeugten regelmäßig oder unregelmäßig geformten Partikeln bestehen, die gegebenenfalls in mit Haftvermittlern behandelter Form eingesetzt werden.

8. Polymerisierbare Massen nach Ansprüchen 1 bis 7, dadurch gekennzeichnet, daß sie als Komponente d) 0,1 bis 10 Gew.-% Initiatoren, Stabilisatoren, Lichtschutzmittel, Pigmente, Farbstoffe, Fluoreszenzmittel und/oder Weichmacher enthalten.

9. Verfahren zur Herstellung füllstoffhaltiger, polymerisierbarer Massen, dadurch gekennzeichnet, daß man
a) 15 bis 60 Gew.-% Monomere mit radikalisch polymerisierbaren Doppelbindungen,
b) 8 bis 40 Gew.-% faserförmige, anorganische Füllstoffe mit einer mittleren Faserlänge von 10 nm bis 300 µm und einem Verhältnis von Länge zu Durchmesser von 2:1 bis 300:1,
c) 15 bis 60 Gew.-% nicht-faserförmige, anorganische Füllstoffe mit einer mittleren Teilchengröße von 10 nm bis 200 µm und
d) 0 bis 10 Gew.-% Hilfsmittel
wobei die Summe a) plus b) plus c) plus d) 100 Gew.-% ergibt, miteinander vermischt.

10. Verwendung von füllstoffhaltigen, polymerisierbaren Massen, die
a) 15 bis 60 Gew.-% Monomere mit radikalisch polymerisierbaren Doppelbindungen,
b) 8 bis 40 Gew.-% faserförmige, anorganische Füllstoffe mit einer mittleren Faserlänge von 10 nm bis 300 µm und einem Verhältnis von Länge zu Durchmesser von 2:1 bis 300:1,
c) 15 bis 60 Gew.-% nicht-faserförmige, anorganische Füllstoffe mit einer mittleren Teilchengröße von 10

nm bis 200 µm und

d) 0 bis 10 Gew.-% Hilfsmittel

enthalten, wobei die Summe a) plus b) plus c) plus d) 100 Gew.-% ergibt,

zur Herstellung von biokompatiblen Werkstoffen für medizinische und zahnmedizinische Anwendungen.